# EUROPEAN PATENT APPLICATION

(11) **EP 4 715 040 A1**
(43) Date of publication of application: **25.03.2026**
(21) Application number: 25817734.4
(22) Date of filing: 13.05.2025
(51) Int. Cl.: C12N 1/20, A61K 47/02, C12N 1/12, A61P 3/04, A61K 31/47, C12N 9/10

(54) **AKK11 STRAIN-CONTAINING PROBIOTIC AGENT CAPABLE OF RELIEVING HYPERURICEMIA, AND THE USE THEREOF**

(30) Priority: 04.06.2024 CN 202410711433
(71) Applicant: Wecare Probiotics Co., Ltd., Suzhou, Jiangsu 215200 (CN)
(72) Inventor: FANG, Shuguang, Suzhou, Jiangsu 215200 (CN); WU, Zhiyi, Suzhou, Jiangsu 215200 (CN); DONG, Yao, Suzhou, Jiangsu 215200 (CN); WANG, Xiaowen, Suzhou, Jiangsu 215200 (CN); GAI, Zhonghui, Suzhou, Jiangsu 215200 (CN); ZHU, Jianguo, Suzhou, Jiangsu 215200 (CN)
(74) Representative: Prinz & Partner mbB
(86) International application number: PCT/CN2025/094534
(87) International publication number: WO 2025/251848

(57) **Abstract**

An Akk11 strain-containing probiotic agent capable of relieving hyperuricemia, and the use thereof. Strains in the probiotic agent include an Akkermansia muciniphila Akk11 strain and a Lactobacillus johnsonii LJ09 strain. It is found in the present application that the two strains possess a potential interaction and can cooperate with each other, thereby synergizing the effect of relieving hyperuricemia, which is specifically manifested in: being capable of remarkably regulating uric acid metabolism and improving renal clearance functions; being capable of effectively inhibiting the activity of a key enzyme for uric acid synthesis so as to reduce the production of uric acid, and being capable of remarkably relieving renal damage and renal dysfunctions associated with high levels of uric acid.

## Description

### TECHNICAL FIELD

The present application belongs to the technical field of probiotic preparations, and relates to a probiotic preparation containing an Akk11 strain for alleviating high uric acid and a use thereof.

### BACKGROUND

Hyperuricemia is a common metabolism disorder disease characterized by increased serum uric acid levels, further resulting in health problems such as gout, kidney stones and kidney failure. Uric acid, which is a waste produced after the human body metabolizes purine, is mainly excreted by the kidneys and partially excreted by the intestinal tracts. Traditional treatment methods focus on using drugs, such as diuretics and uric acid inhibitors, to inhibit the production of uric acid or increase the excretion of uric acid. However, long-term use may be accompanied by the problems of side effects and drug resistance.

Intestinal microecology is closely related to the health of the host, and the balance state of intestinal flora has a potential effect on the prevention and treatment of metabolic diseases. As an effective means to regulate intestinal flora, probiotics have been used for improving metabolic diseases such as obesity and diabetes. Particular probiotic strains may have a positive effect on the production and excretion mechanisms of uric acid by regulating the intestinal environment and the host's metabolic pathway.

However, there are relatively few strategies for preventing and treating hyperuricemia using probiotics in the existing art. In view of this, it is of great significance to develop a treatment plan based on probiotics that can not only effectively reduce the uric acid level but also reduce the need to rely on traditional drug treatment.

### SUMMARY

The present application provides a probiotic preparation containing an Akk11 strain for alleviating high uric acid and a use thereof, and in particular, provides a probiotic preparation containing an Akk11 strain for alleviating high uric acid and a use of the probiotic preparation for preparing a drug for preventing, alleviating or treating hyperuricemia.

In a first aspect, the present application provides a probiotic preparation containing an Akk11 strain for alleviating high uric acid including an *Akkermansia muciniphila* Akk11 strain with a deposit number CCTCC NO: M2024119 and a *Lactobacillus johnsonii* LJ09 strain with a deposit number CGMCC No. 20123.

The *Akkermansia muciniphila* Akk11 strain is deposited in the China Center for Type Culture Collection on January 15, 2024, with a deposit number CCTCC NO: M2024119, where the China Center for Type Culture Collection is located at Wuhan University, Wuhan, China. The *Lactobacillus johnsonii* LJ09 strain is deposited in the China General Microbiological Culture Collection Center on June 22, 2020, with a deposit number CGMCC No. 20123, where the China General Microbiological Culture Collection Center is located at No. 3, NO. 1 West Beichen Road, Chaoyang District, Beijing.

The present application develops a brand-new probiotic compounding manner, which is to compound the *Akkermansia muciniphila* Akk11 strain and the *Lactobacillus johnsonii* LJ09 strain. It is found that there is a potential interaction between the two strains and the two strains can cooperate with each other to exert a synergistic effect in alleviating hyperuricemia, which is specifically manifested by: (1) significantly regulating the uric acid metabolism and improving the renal clearance function; (2) effectively inhibiting the activity of key enzymes for the synthesis of uric acid, thereby reducing the production of uric acid; and (3) significantly alleviating kidney damage and renal dysfunction related to high uric acid levels.

In the case where amounts of bacteria used are consistent, compared with a single Akk11 strain or a single LJ09 strain, the compounding of the two bacteria significantly improves the above efficacy. Therefore, the probiotic preparation provides a new strategy for preventing, alleviating or treating hyperuricemia. Since *Akkermansia muciniphila* and *Lactobacillus johnsonii* are both probiotics, a product with related efficacy prepared from *Akkermansia muciniphila* and *Lactobacillus johnsonii* has high safety and is not easy to develop dependency.

Preferably, a ratio of viable bacterial count of the *Akkermansia muciniphila* Akk11 strain to the viable bacterial count of the *Lactobacillus johnsonii* LJ09 strain is 1:10 to 10:1, for example, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1 or 10:1. Other specific point values within the numerical range may be selected, and details are not described here.

Preferably, the total viable bacterial count in the probiotic preparation is not less than 1×10⁸ CFU/mL or 1×10⁸ CFU/g, for example, 1×10⁸ CFU/mL (CFU/g), 1×10⁹ CFU/mL (CFU/g), 5×10⁹ CFU/mL (CFU/g), 1×10¹⁰ CFU/mL (CFU/g), 5×10¹⁰ CFU/mL (CFU/g), 1×10¹¹ CFU/mL (CFU/g), 1×10¹² CFU/mL (CFU/g) or 1×10¹³ CFU/mL (CFU/g). Other specific point values within the numerical range may be selected, and details are not described here.

Preferably, a dosage form of the probiotic preparation includes a solution, a lyophilized powder, a capsule, a tablet or a granule. The dosage form of the probiotic preparation to which the present application relates is not limited and includes the most commonly used solution and lyophilized powder or further prepared capsule, tablet or granule.

Preferably, the dosage form of the probiotic preparation is the solution, and the solution is prepared through the following method:

inoculating an Akk11 strain and an LJ09 strain in mediums, respectively, for activation and fermentation culture to obtain fermentation broths; separately centrifuging the fermentation broths, and resuspending the fermentation broths with a solvent to obtain an Akk11 bacterial suspension and an LJ09 bacterial suspension; and mixing the Akk11 bacterial suspension with the LJ09 bacterial suspension according to a ratio of viable bacterial counts to obtain the probiotic preparation.

Preferably, the dosage form of the probiotic preparation is the lyophilized powder, and the lyophilized powder is prepared through the following method:
inoculating an Akk11 strain and an LJ09 strain in mediums, respectively, for activation and fermentation culture to obtain fermentation broths; separately centrifuging the fermentation broths, mixing with a protective agent, and then performing lyophilization to obtain Akk11 bacteria powder and LJ09 bacteria powder; and mixing the Akk11 bacteria powder with the LJ09 bacteria powder according to a ratio of viable bacterial counts to obtain the probiotic preparation.

Preferably, the protective agent is selected from any one or a combination of at least two of skim milk, gelatin, dextrin, arabic gum, dextran, sodium alginate, polyvinylpyrrolidone, sucrose, lactose, trehalose, sorbitol or xylitol.

In a second aspect, the present application provides a use of the probiotic preparation of the first aspect for preparing a drug for preventing, alleviating or treating hyperuricemia.

Preferably, the drug further includes an adjuvant, and the adjuvant is selected from any one or a combination of at least two of a filler, a binder, a wetting agent, a disintegrating agent, an emulsifier, a cosolvent, a solubilizer, an osmotic pressure regulator, a colorant, a pH adjusting agent, an anti-oxidant, a bacteriostatic agent or a buffer.

Preferably, the drug further includes a functional additive, and the functional additive is selected from any one or a combination of at least two of fructooligosaccharides, galactooligosaccharides, xylooligosaccharides, inulin, Krestin, polydextrose, α-whey protein or lactoferrin.

Compared with the existing art, the present application has the beneficial effects below.

The present application develops a brand-new probiotic compounding manner, which is to compound the *Akkermansia muciniphila* Akk11 strain and the *Lactobacillus johnsonii* LJ09 strain. It is found that there is a potential interaction between the two strains and the two strains can cooperate with each other to exert a synergistic effect in alleviating hyperuricemia, which is specifically manifested by: (1) significantly regulating the uric acid metabolism and improving the renal clearance function; (2) effectively inhibiting the activity of key enzymes for the synthesis of uric acid, thereby reducing the production of uric acid; and (3) significantly alleviating kidney damage and renal dysfunction related to high uric acid levels.

In the case where amounts of bacteria used are consistent, compared with a single Akk11 strain or a single LJ09 strain, the compounding of the two bacteria significantly improves the above efficacy. Therefore, the probiotic preparation provides a new strategy for preventing, alleviating or treating hyperuricemia. Since *Akkermansia muciniphila* and *Lactobacillus johnsonii* are both probiotics, a product with related efficacy prepared from *Akkermansia muciniphila* and *Lactobacillus johnsonii* has high safety and is not easy to develop dependency.

### BRIEF DESCRIPTION OF DRAWINGS

FIG. 1 is a chart illustrating the statistical result of the level of urine proteins in urine of each group of mice.
FIG. 2 is a chart illustrating the statistical result of the level of urea nitrogen in urine of each group of mice.
FIG. 3 is a chart illustrating the statistical result of the level of uric acid in urine of each group of mice.
FIG. 4 is a chart illustrating the statistical result of the level of uric acid in serum of each group of mice.
FIG. 5 is a chart illustrating the statistical result of the level of urea nitrogen in serum of each group of mice.
FIG. 6 is a chart illustrating the statistical result of the level of creatinine in serum of each group of mice.
FIG. 7 is a chart illustrating the statistical result of the activity of xanthine oxidase in liver tissues of each group of mice.

### DETAILED DESCRIPTION

Technical solutions of the present application are further described below through examples. It is to be understood by those skilled in the art that the examples are intended to facilitate understanding of the present application and are not to be construed as limiting the present application.

The formulation of the medium involved in the following example is as follows:
MRS medium: 10 g/L peptone, 10 g/L beef extract, 20 g/L glucose, 2 g/L sodium acetate, 5 g/L yeast powder, 2 g/L diammonium hydrogen citrate, 2.6 g/L K₂PO₄·3H₂O, 0.1 g/L MgSO₄·7H₂O, 0.05 g/L MnSO₄, 1 mL/L polysorbate 80 (Tween 80) and 0.5 g/L cysteine hydrochloride.

The taxonomic name of the Akk11 strain involved in the following example is *Akkermansia muciniphila* deposited in the China Center for Type Culture Collection on January 15, 2024, with a deposit number CCTCC NO: M2024119, where the China Center for Type Culture Collection is located at Wuhan University, Wuhan, China.

The taxonomic name of the Akk11 strain involved in the following example is *Lactobacillus johnsonii* deposited in the China General Microbiological Culture Collection Center on June 22, 2020, with a deposit number CGMCC No. 20123, where the China General Microbiological Culture Collection Center is located at No. 3, NO. 1 West Beichen Road, Chaoyang District, Beijing.

The bacterial suspension involved in the following example is prepared by the following method: required strains were inoculated in a liquid medium and cultured for 24 h at 37°C for activation two consecutive times to obtain an activation solution, the activation solution was inoculated in a liquid medium at an inoculum volume of 5% (v/v) and cultured for 24 h at 37°C to obtain a bacterial solution, the bacterial solution was centrifuged at 5000 g for 10 min at 4°C and filtered to obtain strains, and the strains were resuspended with a PBS solution to obtain the bacterial suspension.

The data of the experimental results were statistically analyzed by using ggplot2 in language R. Compared with a control group, ### represents p < 0.001; compared with a model group, *** represents p < 0.001, ** represents p < 0.01, * represents p < 0.05, and NS. represents no significant difference.

### Example

This example explores the ability of strains to alleviate symptoms of a high-uric acid mouse model.
(1) Test animals: healthy male SPF ICR mice (70 mice, body mass: 20±2 g, purchased from Shanghai Chengxi Biotechnology Co., Ltd.). These mice were reared in a controlled environment with room temperature maintained at 20-24°C and a humidity of 50% to 60%, following a 12 h light/dark cycle. They had free access to food and water.
(2) Animal grouping: the mice were adaptively fed for 1 week and randomly divided into 7 groups (10 mice for each group): a control group (CTL), a high-uric acid model group (an MC group), an Akk11 strain group (an Akk11 group, recorded as an S1 group), an LJ09 strain group (an LJ09 group, recorded as an S2 group), a commercially available *Akkermansia muciniphila* group (a BNCC341917 group, recorded as an S3 group), a composite probiotic group 1 (an Akk11+LJ09 group with a ratio of viable bacterial counts of 2:1, recorded as an S4 group) and a composite probiotic group 2 (a BNCC341917+LJ09 group with a ratio of viable bacterial counts of 2:1, recorded as an S5 group).
(3) Animal modeling and intervention methods: the mice in the CTL group were first administered with an aqueous solution of 1% sodium carboxymethyl cellulose (CMC-Na, purchased from Shanghai Shenguang Edible Chemicals Co., Ltd.) via gavage and then administered with normal saline via gavage 1 hour later; and other groups were first administered with 270 mg/kg potassium oxonate (suspended in an aqueous solution of 1% CMC-Na, Shanghai Macklin Biochemical Technology Co., Ltd.) via gavage every day and then administered with normal saline (the MC group) or their respective groups of probiotic bacterial solutions (dosage: 10⁸ CFU/mouse/day, the S1 to S5 groups) via gavage 1 hour later for 21 consecutive days.
(4) Indicator analysis

### (4.1) Evaluation of renal functions of high-uric acid mice

Urine from the mice was collected through reflex against stimulation 1 day before the last administration for subsequent 24-hour urinalysis. A specific method includes: measuring the content of urine proteins (UPs) through a Coomassie brilliant blue method, measuring the content of urine urea nitrogen (UUN) through a urease method, and measuring the content of uric acid (UA) through enzyme colorimetry.

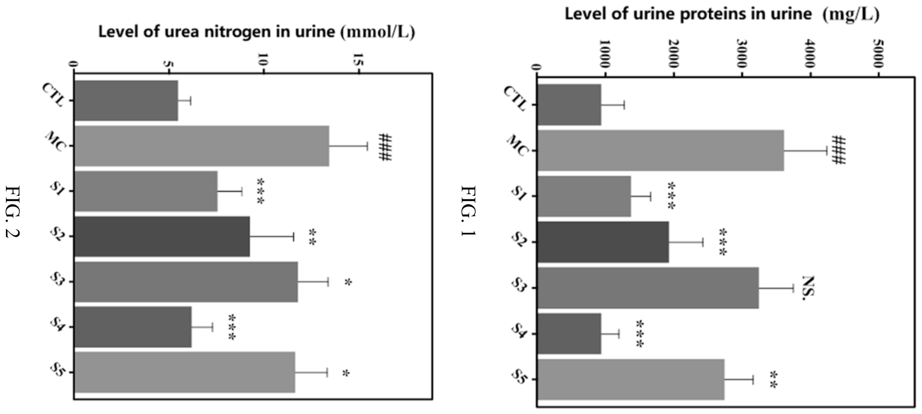

The results are shown in FIGS. 1, 2 and 3. Compared with the CTL group, the levels of urine proteins and urea nitrogen in the urine of the mice in the MC group are both significantly increased, indicating that renal functions are impaired and metabolites are excreted abnormally. Different from the urine proteins and the urea nitrogen, the level of uric acid is decreased significantly in the model group, which may be due to the accumulation of uric acid in blood, reflecting that the ability of kidneys to excrete uric acid is decreased. However, after the intervention of the probiotics, a significant decrease in the levels of urine proteins and urea nitrogen in the urine was observed, indicating that the probiotics has a potential effect in improving renal function damage. In addition, the intervention of the probiotics also significantly increases the level of uric acid in the urine, especially in the S4 group, which may indicate that the probiotic preparation involved in the present application helped restore the uric acid excretion function of the kidneys and reduce the accumulation of uric acid in the blood.

### (4.2) Effects on key biochemical indicators of high uric-acid mice

After the intervention, 1 hour after the administration, blood was collected from each group of mice through eyeball removal. The blood was left standing still for 1 h and centrifuged for 10 min to separate serum. The levels of uric acid, urea nitrogen and creatinine in the serum of the mice were measured through an enzyme-linked immunosorbent assay (ELISA) method by using a kit.

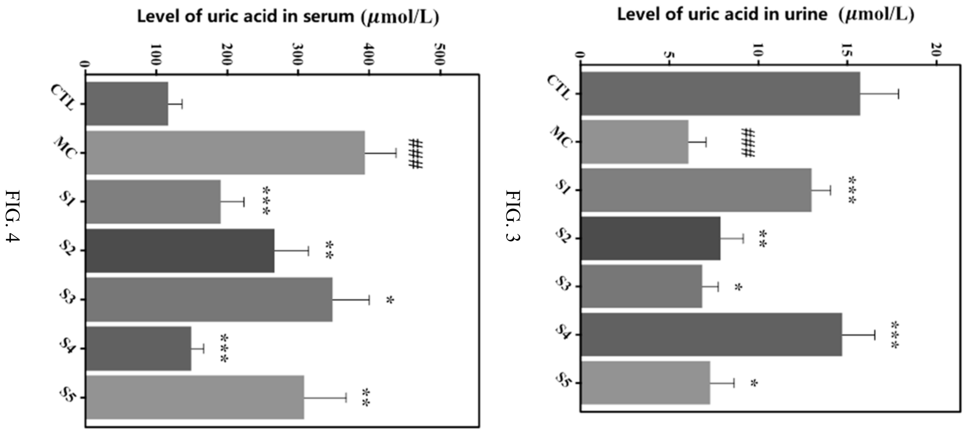

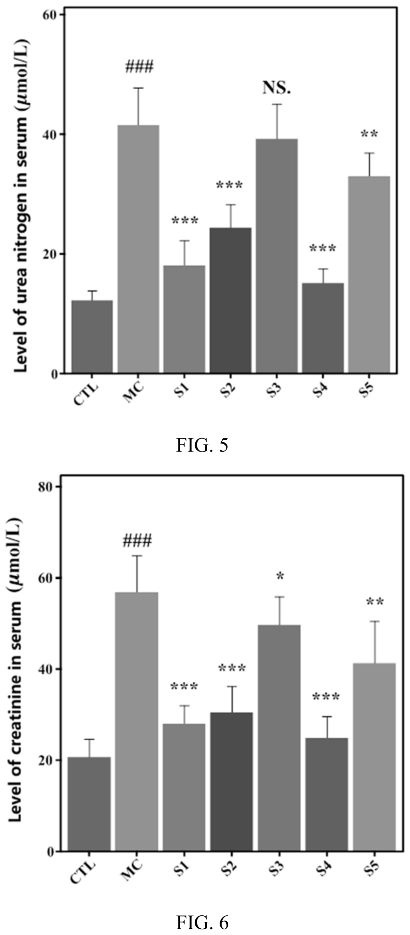

The results are shown in FIGS. 4, 5 and 6. Compared with the CTL group, the levels of uric acid, urea nitrogen and creatinine in the serum of the mice in the MC group are all significantly increased. This phenomenon indicates that the model successfully replicated hyperuricemia and hyperuricemia-related kidney damage. However, after the intervention of the probiotics, the levels of uric acid, urea nitrogen and creatinine in the serum of the mice are all significantly decreased, tending to the levels of the normal control group, especially in the S4 group. This result not only verifies that the probiotic preparation involved in the present application has a potential effect in regulating the level of uric acid, but also implies that the probiotic preparation has a positive effect in protecting the renal functions and promoting the elimination of metabolic wastes.

### (4.3) Effects on activity of xanthine oxidase in livers of high-uric acid mice

The liver is one of the organs with the highest activity of xanthine oxidase (XOD), making the liver an ideal tissue for evaluating the production of uric acid and the metabolism of purine. Through the measurement of the activity of xanthine oxidase in the liver, the systemic production of uric acid can be accurately evaluated, which is of key significance for studying physiological and pathological mechanisms of uric acid-related diseases.

After the intervention was finished, the mice were sacrificed and dissected, and intact livers were taken. After the intact livers were washed with normal saline and wiped dry, the liver homogenate of each group of mice was prepared to obtain supernatant, and the activity of xanthine oxidase (XOD) in the livers of the mice was measured through an enzyme-linked immunosorbent assay (ELISA) method.

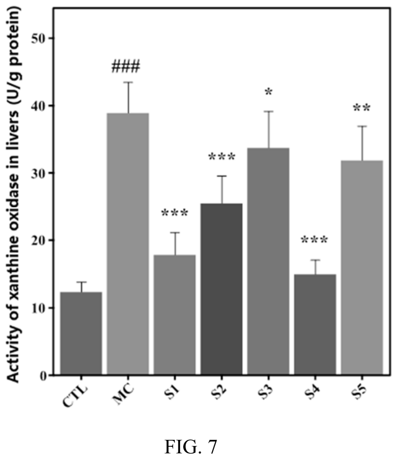

The results are shown in FIG. 7. Compared with the CTL group, the activity of xanthine oxidase (XOD) in the livers of the mice in the MC group is significantly increased, reflecting that the production of uric acid is increased *in vivo,* which is a key marker of hyperuricemia and hyperuricemia-related metabolism disorders. After the intervention of the probiotics, the activity of xanthine oxidase of the mice is significantly decreased, indicating a potential regulatory effect of the probiotics on reducing the production of uric acid, especially in the S4 group. This significant decrease in the activity may be related to an effect of the probiotics on intestinal microbial communities and metabolites of the intestinal microbial communities, thereby indirectly regulating the metabolic function of the liver and reducing the production of uric acid.

The applicant has stated that although the technical solutions of the present application are described through the preceding embodiments, the present application is not limited to the preceding embodiments, which means that the implementation of the present application does not necessarily depend on the preceding embodiments. It is to be understood by those skilled in the art that any improvements made to the present application and equivalent replacements of raw materials of the product, additions of adjuvant ingredients, selections of specific manners, etc. in the present application all fall within the scope of the present application.

Although preferred embodiments of the present application are described above in detail, the present application is not limited to details of the preceding embodiments, and various simple variations can be made to the technical solutions of the present application without departing from the technical concept of the present application. These simple variations are all within the scope of the present application.

Additionally, it is to be noted that if not in collision, specific technical features described in the preceding embodiments can be combined in any suitable manner. To avoid unnecessary repetition, various possible combinations are no longer described in the present application.

## Claims

1. A probiotic preparation containing an Akk11 strain for alleviating high uric acid, comprising an *Akkermansia muciniphila* Akk11 strain with a deposit number CCTCC NO: M2024119 and a *Lactobacillus johnsonii* LJ09 strain with a deposit number CGMCC No. 20123.

2. The probiotic preparation according to claim 1, wherein a ratio of viable bacterial count of the *Akkermansia muciniphila* Akk11 strain to viable bacterial count of the *Lactobacillus johnsonii* LJ09 strain is 1:10 to 10:1.

3. The probiotic preparation according to claim 1, wherein a total viable bacterial count in the probiotic preparation is not less than 1×10⁸ CFU/mL or 1×10⁸ CFU/g.

4. The probiotic preparation according to claim 1, wherein a dosage form of the probiotic preparation comprises a solution, a lyophilized powder, a capsule, a tablet or a granule.

5. The probiotic preparation according to claim 4, wherein the dosage form of the probiotic preparation is the solution, and the solution is prepared through the following method:
inoculating an Akk11 strain and an LJ09 strain in mediums, respectively, for activation and fermentation culture to obtain fermentation broths; separately centrifuging the fermentation broths, and resuspending the fermentation broths with a solvent to obtain an Akk11 bacterial suspension and an LJ09 bacterial suspension; and mixing the Akk11 bacterial suspension with the LJ09 bacterial suspension according to a ratio of viable bacterial counts to obtain the probiotic preparation.

6. The probiotic preparation according to claim 4, wherein the dosage form of the probiotic preparation is the lyophilized powder, and the lyophilized powder is prepared through the following method:
inoculating an Akk11 strain and an LJ09 strain in mediums, respectively, for activation and fermentation culture to obtain fermentation broths; separately centrifuging the fermentation broths, mixing with a protective agent, and then performing lyophilization to obtain Akk11 bacteria powder and LJ09 bacteria powder; and mixing the Akk11 bacteria powder with the LJ09 bacteria powder according to a ratio of viable bacterial counts to obtain the probiotic preparation.

7. The probiotic preparation according to claim 6, wherein the protective agent is selected from any one or a combination of at least two of skim milk, gelatin, dextrin, arabic gum, dextran, sodium alginate, polyvinylpyrrolidone, sucrose, lactose, trehalose, sorbitol or xylitol.

8. A use of the probiotic preparation according to any one of claims 1 to 7 for preparing a drug for preventing, alleviating or treating hyperuricemia.

9. The use according to claim 8, wherein the drug further comprises an adjuvant, and the adjuvant is selected from any one or a combination of at least two of a filler, a binder, a wetting agent, a disintegrating agent, an emulsifier, a cosolvent, a solubilizer, an osmotic pressure regulator, a colorant, a pH adjusting agent, an anti-oxidant, a bacteriostatic agent or a buffer.

10. The use according to claim 8, wherein the drug further comprises a functional additive, and the functional additive is selected from any one or a combination of at least two of fructooligosaccharides, galactooligosaccharides, xylooligosaccharides, inulin, Krestin, polydextrose, α-whey protein or lactoferrin.
